# EUROPÄISCHE PATENTSCHRIFT

(11) **EP 1 235 569 B1**
(45) Veröffentlichungstag und Bekanntmachung des Hinweises auf die Patenterteilung: **11.02.2004**
(21) Anmeldenummer: 00988591.4
(22) Anmeldetag: 16.11.2000
(51) Int. Cl.: A61K 31/37, A61K 45/06, A61P 7/00

(54) **MEDIKAMENT SOWIE AUFEINANDER ABGESTIMMTE KOMBINATION VON MEDIKAMENTEN**
MEDICAMENT AND COMBINATION OF COMPATIBLE MEDICAMENTS
MEDICAMENT ET ASSOCIATION DE MEDICAMENTS COMPATIBLES

(30) Priorität: 19.11.1999 DE 19955607
(43) Veröffentlichungstag der Anmeldung: 04.09.2002
(73) Patentinhaber: responsif GmbH, 91056 Erlangen (DE)
(72) Erfinder: BERTLING, Wolf, 91056 Erlangen (DE)
(74) Vertreter: Gassner, Wolfgang, Dr.
(86) Internationale Anmeldenummer: PCT/DE2000/003977
(87) Internationale Veröffentlichungsnummer: WO 2001/037818

(56) Entgegenhaltungen:
- CHLEBOWSKI R T ET AL: "VITAMIN K-3 INHIBITION OF MALIGNANT MURINE CELL GROWTH AND HUMAN TUMOR COLONY FORMATION." CANCER TREAT REP, (1985) 69 (5), 527-532. , XP008000194
- AKMAN S A ET AL: "SYNERGISTIC CYTOTOXICITY BETWEEN MENADIONE AND DICUMAROL VS. MURINE LEUKEMIA L-1210." J PHARMACOL EXP THER, (1987) 240 (2), 486-491. , XP008000195
- PRICE, PAUL A. ET AL: "Vitamin K counteracts the effect of warfarin in liver but not in bone" THROMB. RES. (1987), 46(1), 121-31 , XP008000198
- TAYLOR, CHARLES T. ET AL: "Vitamin K to reverse excessive anticoagulation: a review of the literatur" PHARMACOTHERAPY (1999), 19(12), 1415-1425 , XP008000165

## Beschreibung

Die Erfindung betrifft ein Medikament sowie eine aufeinander abgestimmte Kombination von Medikamenten. Darüber hinaus betrifft die Erfindung die Verwendung eines Wirkstoffs und eines Antagonisten dieses Wirkstoffs.

Nach dem Stand der Technik sind Medikamente mit mindestens einem Wirkstoff bekannt.

Unter einem Wirkstoff versteht man chemische Elemente, chemische Verbindungen und deren Gemische, die in definierter Dosis mit einem Biosystem in Wechselwirkung treten und dadurch eine gewünschte Wirkung auf das System ausüben. Wirkstoffe wirken in einem Biosystem im allgemeinen über spezifische Rezeptoren.

Ein Antagonist ist ein Stoff oder Stoffgemisch, welcher/s der Wirkung eines Wirkstoffs oder der Wirkung eines körpereigenen Stoffs entgegenwirkt.

Es ist bekannt, Antagonisten als Wirkstoffe zu verabreichen, um dadurch ein Biosystem zu beeinflussen. Solche Antagonisten sind z.B. H₁- und H₂-Rezeptorblocker, welche die Wirkung des Histamins hemmen. Antagonisten werden auch verabreicht, um die Wirkung von überdosierten Wirkstoffen aufzuheben. So wird eine Morphin-Vergiftung mit einem Morphin-Antagonisten, wie Naloxon, behandelt.

Es ist auch bekannt, der antikoagulativen Wirkung von Cumarin-Derivaten, wie Phenprocoumon oder Warfarin, durch Vitamin K entgegenzuwirken. Das kann z.B. bei einer Überdosierung von Cumarin-Derivaten oder bei einer Vergiftung mit Cumarin-Derivaten enthaltendem Rattengift angezeigt sein. In Thijsen et al., Br. J. Haematol. 84 (1993), Seiten 681-685 ist die Verabreichung von Vitamin K1 an einen Patienten offenbart, bei dem die Blutgerinnung langfristig mit Phenprocoumon gehemmt war und bei dem Blutungen aufgetreten sind. In Am. J. lin. Nutr. 1987, 45(4), 847 ist Wirkung der gleichzeitigen Einnahme von Warfarin und Vitamin K₁ beschrieben.

Wirkstoffe wirken im Körper üblicherweise ab einer bestimmten therapeutisch wirksamen Konzentration. Die Konzentration eines Wirkstoffs im Blut wird üblicherweise in Form von Blutspiegeln bzw. Plasmaspiegeln angegeben. Bei einer Therapie soll ein bestimmter Bereich von Blutspiegeln nicht unteroder überschritten werden. Diesen Bereich bezeichnet man als "therapeutische Bandbreite". Als nachteilig erweist sich, daß die Blutspiegel der bei der Therapie verabreichten Wirkstoffe mitunter ober- oder unterhalb der therapeutischen Bandbreite liegen. Blutspiegel, die oberhalb der therapeutischen Bandbreite liegen, sind nicht erwünscht, weil sie häufig unerwünschte Nebenwirkungen verursachen. Blutspiegel unterhalb der therapeutischen Bandbreite führen nicht zu der gewünschten therapeutischen Wirkung.

Auch bei innerhalb der therapeutischen Bandbreite des Wirkstoffs liegenden Blutspiegeln ist es möglich, daß der Wirkstoff in seiner Verfügbarkeit oder seiner Wirksamkeit Schwankungen unterliegt. Schwankungen der Wirksamkeit können auftreten, wenn die Wirkung des Wirkstoffs durch weitere Faktoren beeinflußt wird. Die blutzuckersenkende Wirkung von Insulin kann z.B. in Abhängigkeit vom körpereigenen Glukagon schwanken. Eine diätisch verursachte Schwankung der Wirksamkeit ist aus Pedersen et al., J. Intern. Med. 229 (1991), Seiten 517-520 bekannt. Hier wird beschrieben, daß diätisch aufgenommenes Vitamin K die antikoagulative Wirkung von Warfarin beeinflußt. Schwankungen der Verfügbarkeit treten z.B. bei Plasmaprotein-gebundenen Wirkstoffen auf, wenn Faktoren die Freisetzung vom Plasmaprotein beeinflussen. So wird z.B. ein an Plasmaprotein gebundenes Cumarin-Derivat durch Sulfonamide von seiner Bindungsstelle am Plasmaprotein verdrängt. Dadurch steigt bei konstanten Blutspiegeln die Verfügbarkeit der Cumarin-Derivate bei gleichzeitiger Behandlung mit Sulfonamiden an.

Das Unter- oder Überschreiten der therapeutischen Bandbreite sowie die Schwankungen der Verfügbarkeit und der Wirksamkeit können diätisch, physiologisch-metabolisch, physisch-endogen oder therapeutisch bedingt sein. Die therapeutische Bandbreite, die Verfügbarkeit und die Wirksamkeit eines Medikaments sind außerdem individuell verschieden. Hierbei spielen individuelle Faktoren wie Größe, Körperfettanteil, Metabolisierung etc. eine Rolle.

Aufgabe der Erfindung ist es, die Nachteile nach dem Stand der Technik zu beseitigen. Insbesondere soll ein Medikament oder eine aufeinander abgestimmte Kombination von Medikamenten mit einer möglichst konstanten Wirksamkeit bereitgestellt werden. Der unerwünschte Einfluß diätischer, physiologischmetabolischer, physisch-endogener oder therapeutischer Faktoren auf die Wirksamkeit soll vermindert werden. Das Medikament oder die aufeinander abgestimmte Kombination von Medikamenten soll trotz individueller Unterschiede bei verschiedenen Personen ähnlich wirksam sein. Ziel ist es, die Zuverlässigkeit der therapeutischen Wirkung zu erhöhen.

Diese Aufgabe wird durch die Merkmale der Ansprüche 1 und 5 gelöst. Zweckmäßige Ausgestaltungen der Erfindung ergeben sich aus den Merkmalen der Ansprüche 2 bis 4 und 6 bis 9.

Der Antagonist bewirkt eine Erhöhung der therapeutisch wirksamen Konzentration des Wirkstoffs. Um eine bestimmte therapeutische Wirkung zu erreichen, muß der Wirkstoff also höher dosiert eingesetzt werden als ohne den Antagonisten. Die Auswirkungen von Störfaktoren bzw. individuellen Faktoren auf die Wirksamkeit des in erhöhter Dosierung verabreichten Wirkstoffs sind anteilsmäßig geringer als bei einem herkömmlich dosierten Wirkstoff. Je höher der Antagonist dosiert ist, desto höher liegt die therapeutisch wirksame Konzentration des Wirkstoffs und desto geringer ist der Einfluß von Störfaktoren bzw. individuellen Faktoren auf die Wirksamkeit des Wirkstoffs. Mit dem erfindungsgemäßen Medikament bzw. der aufeinander abgestimmten Kombination von Medikamenten wird im Vergleich zu konventionellen Medikamenten mit größerer Sicherheit die gewünschte therapeutische Wirkung erreicht. Der Patient ist bei einer Therapie mit dem erfindungsgemäßen Medikament weniger eingeschränkt. So muß er sich z.B. weniger streng an Diäten halten, als bei der Therapie mit herkömmlichen Medikamenten. Für den Arzt ist die Medikation mit einem erfindungsgemäßen Medikament einfacher als mit einem herkömmlichen Medikament, weil er individuelle Unterschiede zwischen verschiedenen Patienten weniger stark beachten muß. Das erfindungsgemäße Medikament eröffnet darüber hinaus neue therapeutische Möglichkeiten. Der Einfluß weiterer Medikamente auf das erfindungsgemäße Medikament ist geringer als bei herkömmlichen Medikamenten. Der Arzt kann Wirkstoffe miteinander kombinieren, die bei herkömmlicher Medikation nicht zusammen verabreicht werden können.

Beim erfindungsgemäßen Medikament bzw. der erfindungsgemäß aufeinander abgestimmten.Kombination von Medikamenten liegen der Wirkstoff und der Antagonist in solchen Mengen vor, daß bei einer Therapie eine therapeutisch wirksame Konzentration des Wirkstoffs erreicht wird. Die Wahl geeigneter Mengen und damit eines bestimmten Mengenverhältnisses von Wirkstoff zu Antagonist ermöglicht die Bestimmung der Wirksamkeit des erfindungsgemäßen Medikaments. Erfindungsgemäße Medikamente mit identischer Kombination von Wirkstoff und Antagonist können für unterschiedliche Anwendungen mit verschiedenen Wirksamkeiten bereitgestellt werden.

Beim erfindungsgemäßen Medikament bzw. der erfindungsgemäß aufeinander abgestimmten Kombination von Medikamenten liegt der Antagonist in einer Menge vor, die bei einer Therapie die therapeutisch wirksame Konzentration des Wirkstoffs soweit erhöht, daß die Wirkung des Wirkstoffs durch äußere oder körpereigene Störgrößen oder individuelle Faktoren weitgehend unbeeinflußbar ist. Die Beeinflußbarkeit durch die genannten Störgrößen bzw. Faktoren ist um so geringer, je höher die therapeutisch wirksame Konzentration des Wirkstoffs ist. Die Erhöhung der therapeutisch wirksamen Konzentration des Wirkstoffs bis zur weitgehenden Unbeeinflußbarkeit seiner Wirkung durch Störgrößen bzw. individuelle Faktoren steigert die Zuverlässigkeit des Medikaments.

Bei einer bevorzugten Ausgestaltung des erfindungsgemäßen Medikaments bzw. der erfindungsgemäßen aufeinander abgestimmten Kombination von Medikamenten weist der Wirkstoff zu einem seiner spezifischen Bindungspartner am Wirkort eine andere Affinität auf als der Antagonist zu einem seiner spezifischen Bindungspartner. Dabei können der spezifische Bindungspartner des Wirkstoffs und derjenige des Antagonisten identisch sein.

Das ist beispielsweise dann der Fall, wenn der Wirkstoff und der Antagonist um die Bindung an ein und demselben Rezeptor konkurrieren. Die unterschiedlichen Affinitäten erlauben die Einstellung eines Gleichgewichts zwischen Antagonist und Wirkstoff, welches den Einfluß von Störgrößen "abpuffern" kann. In einem durch ein erfindungsgemäßes Medikament beeinflußten System, in dem z.B. der Antagonist eine höhere Affinität zu dem Rezeptor des Wirkstoffs aufweist als der Wirkstoff selbst, liegt der Wirkstoff hoch dosiert vor. Nur ein ausreichend hoch dosierter Wirkstoff kann einen solchen Antagonisten vom Rezeptor verdrängen und dadurch seine Wirkung entfalten. Kommt zu diesem System z.B. ein körpereigener Störfaktor hinzu, der mit ähnlicher Affinität wie der Wirkstoff um die Bindung an demselben Rezeptor konkurriert, so ist dessen Einfluß auf das System wegen des hoch dosierten Wirkstoffs gering.

Bei einer weiteren Ausgestaltung ist als Antagonist Vitamin K und als Wirkstoff mindestens ein Vitamin K-Antagonist enthalten. Der Vitamin K-Antagonist kann dabei aus der Gruppe der Cumarin-Derivate ausgewählt sein. Dies sind z.B. Dicoumarol, Phenprocoumon (Marcumar®), Acenocoumarol (Sintrom®) und Warfarin (Coumadin®). Bevorzugt enthält ein erfindungsgemäßes Medikament 0,1 bis 10 mg Vitamin K und 3 bis 10 mg Phenprocoumon, vorzugsweise 0,5 bis 3 mg Vitamin K und 4,5 bis 7,5 mg Phenprocoumon, insbesondere 0,5 bis 1,5 mg Vitamin K und 5 bis 7 mg Phenprocoumon. Das Medikament kann ein bis vier mal täglich verabreicht werden.

Die Erfindung betrifft außerdem die Verwendung eines Wirkstoffs und eines Antagonisten dieses Wirkstoffs zur Herstellung eines Medikaments oder einer aufeinander abgestimmten Kombination von Medikamenten zur kombinierten Verabreichung.

Die Erfindung umfaßt auch die Therapie eines Patienten durch Verabreichung des Medikaments oder einer entsprechend hergerichteten Kombination von Medikamenten. Das Medikament oder die Kombination von Medikamenten ist dabei mit einer schriftlichen Anweisung für den Patienten versehen; die schriftliche Anweisung enthält im Falle der Kombination Angaben über die Mengen und den maximalen zeitlichen Abstand der einzunehmenden Medikamente. Die Erfindung umfaßt ferner einen Stoff, in dem mindestens ein therapeutischer Wirkstoff und mindestens ein Antagonist dieses Wirkstoffs enthalten ist. Hinsichtlich der vorteilhaften Ausgestaltungen des Wirkstoffs und des Stoffs wird auf die vorangegangenen Ausführungen verwiesen.

Die genannten und die nachstehend noch zu erläuternden Merkmale sind nicht nur in den jeweils angegebenen Kombinationen, sondern auch in anderen Kombinationen oder in Alleinstellung verwendbar. Weitere Vorteile ergeben sich aus folgendem Ausführungsbeispiel und im Zusammenhang mit der Zeichnung. Hierin zeigen
- Fig. 1: eine schematische Darstellung der Wirkung eines Cumarin-Derivats auf den Vitamin K-Kreislauf und auf die Blutgerinnung,
- Fig. 2: eine schematische Darstellung der therapeutischen Bandbreite und des Plasmaspiegels von Phenprocoumon sowie der Schwankungen der antikoagulativen Wirkung von Phenprocoumon bei Verabreichung mit einem Medikament nach dem Stand der Technik und
- Fig. 3: eine schematische Darstellung der therapeutischen Bandbreite und des Plasmaspiegels von Phenprocoumon sowie der Schwankungen der antikoagulativen Wirkung von Phenprocoumon bei Verabreichung mit einem erfindungsgemäßen Medikament.

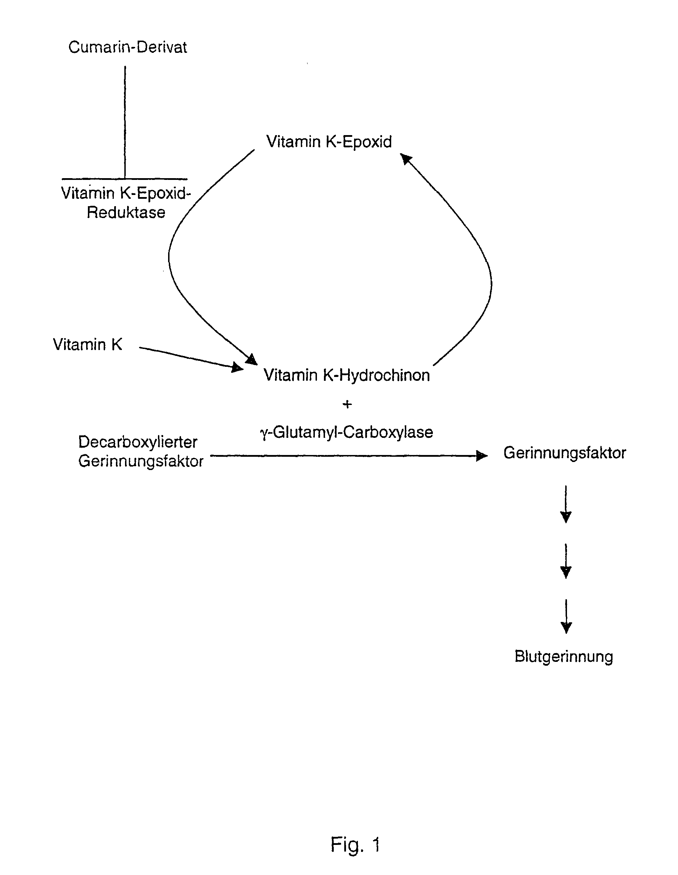

Die in Fig. 1 ausgewiesenen decarboxylierten Gerinnungsfaktoren sind solche Gerinnungsfaktoren, die durch Carboxylierung aktiviert werden. Sie werden durch die Aktivierung in die Lage versetzt, eine Blutgerinnung auszulösen. Als decarboxylierte Gerinnungsfaktoren können vorliegen: Faktor VII, Protein C, Faktor IX, Protein S, Faktor X und Prothrombin. Die Carboxylierung wird durch das Enzym γ-Glutamyl-Carboxylase katalysiert. Vitamin K-Hydrochinon wird dabei als Cofaktor in stöchiometrischen Mengen zu Vitamin K-Epoxid umgesetzt. Der größte Teil des Vitamin K-Hydrochinons wird aus Vitamin K-Epoxid mit Hilfe des Enzyms Vitamin K-Epoxid-Reduktase (VKER) wiedergewonnen. Weniger als 1 % des Vitamin K-Hydrochinons wird aus neu aufgenommenem Vitamin K gebildet. Der tägliche Vitamin K-Bedarf beträgt 0,03 - 1,5 µg/kg-Körpergewicht. VKER kann durch Cumarin-Derivate, wie Warfarin oder Phenprocoumon, gehemmt werden. Die resultierende Hemmung der Vitamin K-Hydrochinon-Produktion und damit der Carboxylierung der decarboxylierten Gerinnungsfaktoren führt zur Hemmung der Blutgerinnung. Bei einer ausreichend hohen Dosierung von Cumarin-Derivaten kann die Wiedergewinnung von Vitamin K-Hydrochinon aus Vitamin K-Epoxid weitgehend unterbunden werden. Die Vitamin K-Hydrochinon-Produktion und die Blutgerinnung hängen dann vorwiegend vom zugeführten Vitamin K ab.

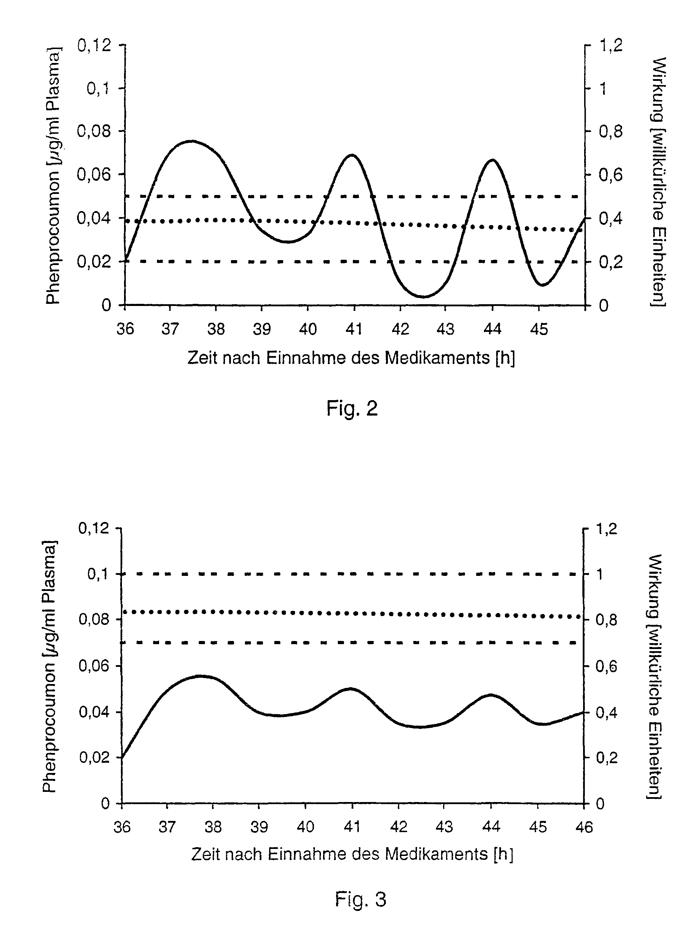

Vitamin K ist in Lebensmitteln in unterschiedlichen Mengen vorhanden. Die blutgerinnungshemmende Wirkung von Cumarin-Derivaten schwankt in Abhängigkeit von der mit der Nahrung aufgenommenen Vitamin K-Menge. Das ist beispielhaft für den mit einem herkömmlichen Medikament verabreichten Wirkstoff Phenprocoumon in Fig. 2 gezeigt. Die Grenzen der therapeutischen Bandbreite von Phenprocoumon sind hier durch gestrichelte Linien darstellt. Sie liegt im Bereich zwischen 0,02 und 0,05 µg Phenprocoumon pro ml Plasma. Nach der Einnahme des Medikaments stellt sich ein durch die gepunktete Linie gezeigter Phenprocoumon-Plasmaspiegel innerhalb der therapeutischen Bandbreite ein. Der Plasmaspiegel ist auf der links angeordneten Y-Achse skaliert. Die blutgerinnungshemmende Wirkung ist durch die durchgehende Linie dargestellt. Sie ist in willkürlichen Einheiten angegeben, die auf der rechts angeordneten Y-Achse skaliert sind. Da im Körper zunächst noch carboxylierte Gerinnungsfaktoren vorliegen, setzt die blutgerinnungshemmende Wirkung erst nach deren Verbrauch ein. Die Latenzzeit beträgt ca. 6 Stunden, die volle Wirkung tritt erst nach 36 bis 72 Stunden ein. Die blutgerinnungshemmende Wirkung bleibt jedoch nicht wie der Plasmaspiegel relativ konstant. Der hier dargestellte, wiederholte starke Abfall der blutgerinnungshemmenden Wirkung ist durch eine jeweilige Aufnahme von Vitamin K mit der Nahrung bedingt. Die blutgerinnungshemmende Wirkung steigt erst wieder nach dem Sinken des hier nicht dargestellten Plasmaspiegels von Vitamin K an.

Auch in Fig. 3 stellen die gestrichelten Linien die Grenzen der therapeutischen Bandbreite von Phenprocoumon dar. Die gepunktete Linie zeigt den Phenprocoumon-Plasmaspiegel und die durchgehende Linie die blutgerinnungshemmende Wirkung von Phenprocoumon an. Die Skalierung der Achsen ist identisch mit derjenigen in Fig. 2. Bei dem in Fig. 3 dargelegten Beispiel wird mit einem erfindungsgemäßen Medikament neben Phenprocoumon eine bestimmte Menge an Vitamin K zugeführt. Durch das gleichzeitig zugeführte Vitamin K wird die therapeutische Bandbreite von Phenprocoumon zu einem höheren Plasmaspiegel verschoben. Sie liegt hier zwischen 0,07 und 0,1 µg Phenprocoumon pro ml Plasma. In dem erfindungsgemäßen Medikament ist daher im Vergleich zu einem herkömmlichen Phenprocoumon-Medikament eine höhere Menge an Phenprocoumon enthalten. Wegen der weitgehenden Blockade der VKER durch Phenprocoumon ist das mit dem Medikament verabreichte Vitamin K die wesentliche Quelle für die Erzeugung von Vitamin K-Hydrochinon. Es ist so dosiert, daß die Blutgerinnung gehemmt ist aber so weit erhalten bleibt, daß bei einem Patienten keine unerwünschten Blutungen auftreten. Nach der Einnahme des erfindungsgemäßen Medikaments stellt sich ein Phenprocoumon-Plasmaspiegel innerhalb der therapeutischen Bandbreite ein. Die blutgerinnungshemmende Wirkung bleibt auch nach Aufnahme von Vitamin K mit der Nahrung relativ stabil. Die mit der Nahrung zugeführte Vitamin K-Menge stellt nur einen geringen Anteil des insgesamt aufgenommenen Vitamin K dar. Sie wirkt sich somit viel schwächer auf die Hemmung der Blutgerinnung aus als bei einer Therapie mit einem Phenprocoumon-Medikament nach dem Stand der Technik, wie sie in Fig. 2 dargestellt ist. Das erfindungsgemäße Medikament bewirkt eine Stabilisierung der antikoagulativen Wirkung von Phenprocoumon.

## Patentansprüche

1. Medikament mit mindestens einem Wirkstoff, wobei mindestens ein Antagonist dieses Wirkstoffs enthalten ist, wobei der Wirkstoff und der Antagonist in solchen Mengen vorliegen, daß bei einer Therapie eine therapeutisch wirksame Konzentration des Wirkstoffs erreicht wird und daß die Wirkung des Wirkstoffs durch äußere oder körpereigene Störgrößen oder individuelle Faktoren weitgehend unbeeinflußbar ist.

2. Aufeinander abgestimmte Kombination zweier Medikamente, wobei ein erstes Medikament mindestens einen Wirkstoff und ein zweites Medikament mindestens einen Antagonisten dieses Wirkstoffs enthält, wobei der Wirkstoff und der Antagonist in solchen Mengen vorliegen, daß bei einer Therapie eine therapeutisch wirksame Konzentration des Wirkstoffs erreicht wird und daß die Wirkung des Wirkstoffs durch äußere oder körpereigene Störgrößen oder individuelle Faktoren weitgehend unbeeinflußbar ist.

3. Medikament oder aufeinander abgestimmte Kombination von Medikamenten nach Anspruch 1 oder 2, **dadurch gekennzeichnet, daß** der Wirkstoff zu einem seiner spezifischen Bindungspartner am Wirkort eine andere Affinität aufweist als der Antagonist zu einem seiner spezifischen Bindungspartner.

4. Medikament oder aufeinander abgestimmte Kombination von Medikamenten nach einem der Ansprüche 1 bis 3, **dadurch gekennzeichnet, daß** als Antagonist Vitamin K und als wirkstoff mindestens ein Vitamin K-Antagonist, insbesondere ein Cumarin-Derivat, insbesondere Dicoumarol, Phenprocoumon, Acenocoumarol oder Warfarin, enthalten ist.

5. Medikament oder aufeinander abgestimmte Kombination von Medikamenten nach einem der Ansprüche 1 bis 4, **dadurch gekennzeichnet, daß** darin 0,1 bis 10 mg Vitamin K und 3 bis 10 mg Phenprocoumon, vorzugsweise 0,5 bis 3 mg Vitamin K und 4,5 bis 7,5 mg Phenprocoumon, insbesondere 0,5 bis 1,5 mg Vitamin K und 5 bis 7 mg Phenprocoumon, enthalten sind.

6. Verwendung mindestens eines Wirkstoffs und mindestens eines Antagonisten dieses Wirkstoffs zur Herstellung eines Medikaments oder einer aufeinander abgestimmten Kombination von Medikamenten, wobei der Wirkstoff und der Antagonist in solchen Mengen vorliegen, daß bei einer Therapie eine therapeutisch wirksame Konzentration des Wirkstoffs erreicht wird und daß die Wirkung des Wirkstoffs durch äußere oder körpereigene Störgrößen oder individuelle Faktoren weitgehend unbeeinflußbar ist.

7. Verwendung nach Anspruch 6, **dadurch gekennzeichnet, daß** der Wirkstoff zu einem seiner spezifischen Bindungspartner am Wirkort eine andere Affinität aufweist als der Antagonist zu einem seiner spezifischen Bindungspartner.

8. Verwendung nach einem der Ansprüche 6 oder 7, **dadurch gekennzeichnet, daß** der Antagonist Vitamin K und der Wirkstoff mindestens ein Vitamin K-Antagonist, insbesondere ein Cumarin-Derivat, insbesondere Dicoumarol, Phenprocoumon, Acenocoumarol oder Warfarin, ist.

9. Verwendung nach einem der Ansprüche 6 bis 8, **dadurch gekennzeichnet, daß** in dem Medikament oder der aufeinander abgestimmten Kombination von Medikamenten 0,1 bis 10 mg Vitamin K und 3 bis 10 mg Phenprocoumon, vorzugsweise 0,5 bis 3 mg Vitamin K und 4,5 bis 7,5 mg Phenprocoumon, insbesondere 0,5 bis 1,5 mg Vitamin K und 5 bis 7 mg Phenprocoumon, vorliegen.

## Claims

1. A medicament having at least one agent, wherein at least one antagonist of this agent is comprised, the agent and the antagonist being present in amounts such that a therapeutically effective concentration of the agent is reached during therapy and that external or endogenous interfering variables or individual factors are substantially unable to influence the effect of the agent.

2. A mutually co-ordinated combination of two medicaments, where a first medicament comprises at least one agent and a second medicament comprises at least one antagonist of this agent, the agent and the antagonist being present in amounts such that a therapeutically effective concentration of the agent is reached during therapy and that external or endogenous interfering variables or individual factors are substantially unable to influence the effect of the agent.

3. A medicament or mutually co-ordinated combination of medicaments as claimed in any of claims 1 or 2, **characterised in that** the affinity of the agent for its specific binding partner at the site of effect differs from that of the antagonist for its specific binding partner.

4. A medicament or mutually co-ordinated combination of medicaments as claimed in any of claims 1 to 3, **characterised in that** vitamin K is present as antagonist, and at least one vitamin K antagonist, in particular a coumarin derivative, in particular dicoumarol, phenprocoumon, acenocoumarol or warfarin, is present as agent.

5. A medicament or mutually co-ordinated combination of medicaments as claimed in any of claims 1 to 4, **characterised in that** from 0.1 to 10 mg of vitamin K and from 3 to 10 mg of phenprocoumon, preferably 0.5 to 3 mg of vitamin K and 4.5 to 7.5 mg of phenprocoumon, in particular 0.5 to 1.5 mg of vitamin K and 5 to 7 mg of phenprocoumon, are present therein.

6. The use of at least one agent and of at least one antagonist of this agent for producing a medicament or a mutually co-ordinated combination of medicaments, the agent and the antagonist being present in amounts such that a therapeutically effective concentration of the agent is reached during therapy and that external or endogenous interfering variables or individual factors are substantially unable to influence the effect of the agent.

7. The use as claimed in claim 6, **characterised in that** the affinity of the agent for its specific binding partner at the site of effect differs from that of the antagonist for its specific binding partner.

8. The use as claimed in any of claims 6 or 7, **characterised in that** vitamin K is present as antagonist, and at least one vitamin K antagonist, in particular a coumarin derivative, in particular dicoumarol, phenprocoumon, acenocoumarol or warfarin, is present as agent.

9. The use as claimed in any of claims 6 to 8, **characterised in that** from 0.1 to 10 mg of vitamin K and from 3 to 10 mg of phenprocoumon, preferably 0.5 to 3 mg of vitamin K and 4.5 to 7.5 mg of phenprocoumon, in particular 0.5 to 1.5 mg of vitamin K and 5 to 7 mg of phenprocoumon, are present in the medicament or the mutually co-ordinated combination of medicaments.

## Revendications

1. Médicament avec au moins un agent, comprenant au moins un antagoniste de ce agent, le agent et l'antagoniste étant dans de telles quantités qu'une concentration efficace du point de vue thérapeutique du agent soit obtenue lors d'une thérapie et que l'effet du agent ne soit pas, dans une grande mesure, influençable par des facteurs de perturbation externes ou propres au corps, ou bien des facteurs individuels.

2. Une combinaison appropriée de deux médicaments, un premier médicament comprenant au moins un agent et un deuxième médicament au moins un antagoniste de ce agent, le agent et l'antagoniste étant dans de telles quantités qu'une concentration efficace du point de vue thérapeutique du agent soit obtenue lors d'une thérapie et que l'effet du agent ne soit pas, dans une grande mesure, influençable par des facteurs de perturbation externes ou propres au corps, ou bien des facteurs individuels.

3. Un médicament ou une combinaison appropriée de médicaments selon la revendication 1 ou 2, **caractérisé par le fait que** le agent présente, par rapport à un de ses partenaires de liaison spécifiques à la localisation de l'effet, une autre affinité que l'antagoniste par rapport à un des ses partenaires de liaison spécifiques.

4. Un médicament ou une combinaison appropriée de médicaments selon l'une des revendications 1 à 3, **caractérisé par le fait que** sont compris de la vitamine K en tant qu'antagoniste et au moins un antagoniste vitamine K en tant que agent, en particulier un dérivé coumarine, en particulier dicoumarol, phenprocoumon, acenocoumarol ou warfarine.

5. Un médicament ou une combinaison appropriée de médicaments selon l'une des revendications 1 à 4, **caractérisé par le fait que** 0,1 à 10 mg de vitamine K et 3 à 10 mg de phenprocoumon, de préférence 0,5 à 3 mg de vitamine K et 4,5 à 7,5 mg de phenprocoumon, en particulier 0,5 à 1,5 mg de vitamine K et 5 à 7 mg de phenprocoumon, y sont compris.

6. Utilisation d'au moins un agent et d'au moins un antagoniste de ce agent pour la fabrication d'un médicament ou d'une combinaison de médicaments appropriée, le agent et l'antagoniste étant dans de telles quantités que, lors d'une thérapie, une concentration efficace du point de vue thérapeutique du agent soit obtenue et que l'effet du agent ne soit pas, dans une grande mesure, influençable par des facteurs de perturbation externes ou propres au corps, ou bien des facteurs individuels.

7. Utilisation selon la revendication 6, **caractérisé par le fait que** le agent présente, par rapport à un de ses partenaires de liaison spécifiques à la localisation de l'effet, une autre affinité que l'antagoniste par rapport à un de ses partenaires de liaison spécifiques.

8. Utilisation selon l'une des revendications 6 ou 7, **caractérisé par le fait que** l'antagoniste vitamine K et que le agent est au moins un antagoniste vitamine K, en particulier un dérivé coumarine, en particulier dicoumarol, phenprocoumon, acenocoumarol ou warfarine.

9. Utilisation selon l'une des revendications 6 à 8, **caractérisé par le fait que** le médicament ou la combinaison appropriée de médicaments présente 0,1 à 10 mg de vitamine K et 3 à 10 mg de phenprocoumon, de préférence, 0,5 à 3 mg de vitamine K et 4,5 à 7,5 mg de phenprocoumon, en particulier 0,5 à 1,5 mg de vitamine K et 5 à 7 mg de phenprocoumon.
